# EUROPEAN PATENT APPLICATION

(11) **EP 0 896 792 A1**
(43) Date of publication of application: **17.02.1999**
(21) Application number: 97113983.7
(22) Date of filing: 13.08.1997
(51) Int. Cl.: A01N 65/00, A61K 33/26, A61K 31/70

(54) **Antiviral agent**

(71) Applicant: Julphar Pharma GmbH, 37269 Eschwege (DE)
(72) Inventor: Jassim, Sabah A. A. Dr., c/o Julpha Pharma GmbH, 37269 Eschwege (DE)
(74) Representative: Weiss, Wolfgang, Dipl.-Chem. Dr.

(57) **Abstract**

The present invention relates to novel compositions having antiviral and antitumor activity. Further, the invention relates to methods for controlling viruses and for selectively degrading viral nucleic acids.

## Description

The present invention relates to novel compositions having antiviral and antitumor activity. Further, the invention relates to methods for controlling viruses and for selectively degrading viral nucleic acids.

It is important to evaluate and control the presence of viruses and their inactivation from surfaces (e.g. inanimate surfaces, body tissues, nosocomial equipment) and water (e.g. drinking, sewage and sea water). As the detection and use of mammalian viruses can be fastidious, phages (bacterial viruses) represent a diverse group of viruses that exert both positive and negative effects in microbiology and offer potential as an alternative tool for investigating the mechanism of the virucidal action of biocides (Mallard et al. 1996 a, b). In this respect, coliphages and phages infecting Bacteroides fragilis (Kott et al. 1974; Tartera et al. 1988; Dutka et al. 1990; Mesquita 1990; Abad et al. 1994; Armon and Kott 1995; Bosh et al. 1995; Havelarr et al. 1995; Jofre et al. 1995a, b; Araujo et al. 1997) have also been considered as indicators or models of human enteroviral (i.e. poliovirus, human rotaviruses, hepatitis A virus and adenoviruses) contamination because of their resistance to decontamination processes.

Recent developments using phage amplification technology, thermal change and genetically recombinant phages, containing either the bacterial luciferase lux genes or the ice nucleation gene ina, have established an additional value for phage in the rapid detection of bacteria in food and environmental samples (Ulitzur and Kuhn, 1987; Stewart et al., 1989; Jassim et al., 1990; Wolber and Green, 1990; Kodikara et al., 1991; Jassim et al., 1992; Turpin et al., 1993; Jassim et al., 1996; Hibma et al., 1997). In the above examples, the eventual phage destruction is important either from the perspective of maintaining effective fermentation or from the viewpoint of good microbiological practice in the disposal of contaminated material.

Chemical agents play an important role in the disinfection of hospitals and the environment, mainly in connection with water treatment (Quinn, 1992). At present, numerous physical and chemical methods are available for virus, and in particular phage destruction (Adams, 1959; Suzuki et al., 1977; Davidson and Branden, 1981; Fujita et al., 1981; Fujita and Matsuo, 1987; Thurman and Gerba, 1989; Hirotani et al., 1991; Gordon et al., 1993; Maillard et al., 1993; Sagripanti et al., 1993; Maillard et al., 1996 a, b). These methods, however, have a significant impact on the viability and survival of associated host cells. In the case of a sterilization regime this is of little significance but the ability to combat phage in industrial environments would benefit from an environmentally benign procedure that could differentially destroy the virus without damaging metabolically active commercially important bacterial and eukaryotic cells.

It is very well known that nucleic acid is the infectious component of viruses. Its critical importance is underlined by the immunodeficiency virus (HIV), the hepatitis B virus (HBV) and the oncoviruses, whose nucleic acid can become integrated into the host genome (Martin, 1978). Two main targets of biocides are viral proteins and viral nucleic acid. The destruction of the viral capsid and envelope usually suffices to stop viral infection. However, the breakdown of viral nucleic acid would render the virus non-infectious and avoid phenomena such as multiplicity reactivation (Young and Sharp, 1985; Thurman and Gerba, 1989). The destruction of capsids has been demonstrated on numerous occasions with HBV (Thraenhart et al., 1978) and with the Pseudomonas aeruginosa PAO phage F116 (Maillard et al., 1995). Recently, the release and the possible alteration of viral nucleic acid of F116 phage were investigated using a wide range of biocides such as chlorhexidine diacetate (1 % w/v, pH7), cetylpyridinium chloride (0.05 % w/v, pH7), glutaraldehyde (1 % w/v, pH8), ethanol (70 and 100 % v/v), isopropanol (100 % v/v), phenol (1 and 2 % w/v, pH6) and peracetic acid (1 % v/v, pH 4.5) and it was found that the latter significantly affected the phage genome (Maillard et al., 1996b). The studies of SDS polyacrylamide gel electrophoresis (SDS-PAGE) shows that all the above test biocides altered the control band pattern of F116 phage in different ways, but the alteration in the protein band pattern did not always correlate with phage inactivation (Maillard et al., 1996a).

Plant extracts have long proved to be a rich source of antimicrobial and pharmacologically active compounds (Evans, 1989; Carson and Riley, 1992; Phillips, 1992; Shead et al., 1992). Antiviral activity of various plants, fruits and fruit juices has been reported (Chantrill et al., 1952; Thresh, 1956; Konowalchuk and Speirs, 1976a, b, 1978a, b; Hudson, 1990). In these investigations, it has been found that antiviral activity often is associated with tannins. The degree of antiviral activity described in said publications, however, is generally insufficient.

Recently, Jassim et al. (1995) have discovered a new virucidal agent which destroyed phages of various species without damaging metabolically active bacterial cells, by the activation of Fe²⁺ ions with the extracts of either pomegranate rind, Viburnum plicatum leaves or flowers, tea leaves or maple leaves. It was, however, found that the degree of antiviral activity of these agents showed great variations depending on the source of the plant extract and the season. Further, some viruses were inactivated by iron or copper ions alone although associated with damaging, metabolically active bacterial cells (Sagripanti et al., 1993; Jassim et al., 1995).

The object underlying the present invention, therefore, was to provide a composition of antiviral effect which is of enhanced efficacy and reproducibility as compared to the compositions of the prior art.

This object is accomplished by a composition comprising a carrier and an active agent which is a combination of (a) tannic acid or components thereof in a concentration equivalent to 0.003 - 0.3 % (w/v) or in a concentration equivalent to from 30 to 3000 µg/ml tannic acid from Quercus infectoria and (b) a source of Fe²⁺ ions. The given concentration range is based on the total composition weight.

The present invention describes that tannic acid as the antiviral active component of some plants is very effective at a specific preferred concentration range when mixed with a source of Fe²⁺ ions, e.g. a ferrous salt. The invention also includes a method of controlling human viruses such as immunodeficiency virus (HIV), animal viruses such as chicken fowlpox virus, plant viruses such as wheat streak mosaic virus, potato leafroll virus and luteovirus, and all phages of both Gram positive and Gram negative bacteria. This method preferably comprises contacting the viruses with the novel antiviral agent for a time sufficient to inactivate the viruses, e.g. 3 min at room temperature.

The term "tannic acid" as used in the present invention pertains to a composition comprising a complex mixture of naturally occurring polyphenolic compounds which can be divided into two subcategories: hydrolyzable and condensed. The condensed tannins are based on a flavanol structure while the hydrolyzable tannins are simple phenolic acids (gallic acid) linked to sugar (glucose). The hydrolyzable tannins from a given plant extract are usually composed of a mixture of many derivatives of this framework and there is also the possibility that during the extraction process chemical changes in these compounds could occur.

The following hydrolyzable tannins have been specifically isolated from Quercus infectoria (Haslam, 1989):
1. β-1,6-di-O-galloyl-D-glucose
2. β-1,2,3,6-tetra-O-galloyl-D-glucose
3. β-penta-O-galloyl-D-glucose

The following derivatives of these compounds have been isolated from Quercus sp.:
1. tellimagandin II (eugeniin), sometimes called ellagitannin
2. tellimagrandin I
3. casuarictin
4. potentillin
5. pedunculagin
6. vescalagin
7. castalagin

While these compounds have been identified to be in the mixture known as tannic acid or tannin, there are a large number of further compounds which are also present in this mixture. The present invention relates not only to the mixture of these compounds but also to one or several components or fractions of this mixture which have antiviral, antitumor and/or nucleic acid-degrading activity in a concentration range as defined above.

Whereas according to the present invention tannic acid from Quercus infectoria is preferred, tannic acids of any other plants can, of course, also be used, e.g. from oak or chestnut wood, divi-divi, sumach, myrobalanes, trillo, valonea etc. The effective concentration range of tannic acids from these other sources can easily be detected in simple experiments by the person ordinarily skilled in the art.

The given concentration range of from 0.003 to 0.3 % (w/v) corresponds to a concentration of from 30 to 3000 µg/ml and is based on the total composition weight in a liquid aqueous carrier. Since, as described below, any type of carrier can be used, the concentration range may vary when different carriers, e.g. emulsions or solid carriers, are used. The effective concentration range with these carriers, however, can easily be detected in simple experiments by the person ordinarily skilled in the art.

Preferably, the composition of the present invention comprises tannic acids or components thereof in a concentration equivalent to 0.015 to 0.15 % (w/v) tannic acid from Quercus infectoria. Most preferably, the concentration is equivalent to about 0.03 % (w/v) tannic acid from Quercus infectoria.

The source of Fe²⁺ ions is a compound which is capable of generating Fe²⁺ ions in a liquid, preferably an aqueous carrier. Preferably, the source of Fe²⁺ ions is an Fe²⁺ (ferrous) salt. The nature of the salt anion is not critical, examples for suitable salts are Fe-(II)-sulfate, Fe-(II)-halides and organic Fe-(II)-salts. For pharmaceutical purposes, the salt is preferably nontoxic to bacterial or other cells under the intended conditions of use and is water-soluble. A preferred ferrous salt concentration range is 0.1 mM to 0.1 M, more preferably from 1 to 20 mM and more preferably from 2 to 10 mM.

The novel composition comprising tannic acid in combination with ferrous ions has antiviral activity against various species of prokaryotic and eukaryotic viruses, namely phages, human viruses (HIV, herpes simplex HSV-1 and HSV-2 and influenza viruses), animal viruses (chicken fowlpox virus) and plant viruses (potato leafroll virus, wheat streak mosaic virus, luteovirus, tomato virus). Surprisingly, it was found that the antiviral composition does not cause any significant damage to metabolically active host cells such as bacterial, human, animal or plant cells.

In one embodiment of the present invention the carrier is a liquid carrier such as water, an organic solvent such as an alcohol, e.g. ethanol, or a mixture of aqueous and organic solvents. In further embodiments of the present invention the carrier can be an emulsion, e.g. a cream, a solid carrier, e.g. a powder, a gel or any other suitable carrier. For pharmaceutical or agricultural applications in general pharmaceutically or agriculturally acceptable carriers are selected.

It should be pointed out that the composition according to the present invention can be stored in a ready-to-use form, which has the active components in an effective concentration. It is, however, also possible to prepare concentrates of the composition, which can be stored and diluted prior to use.

In general, with any virucidal agents the accepted criterion of virucidal efficacy is a 4.0 log₁₀ reduction in virus infectivity levels which is not always possible to demonstrate, since the major difficulty associated with chemical disinfectants against HIV is the cytotoxic effect of the biocide on the host cells (Gordon et al., 1993). The combination amount of tannic acid and Fe²⁺ ions causes complete destruction (i.e. inactivates viruses by up to ten log₁₀ cycles) of human viruses, animal viruses, plant viruses and bacteria viruses (phage) within 3 min at room temperature. Thus, the antiviral agent can even be used to control human viruses such as HIV and Astro viruses (non-envelope viruses are the most resistant mammalian viruses) with no adverse activity against metabolically active host cells. This provides significant opportunities for the development of a novel virucidal agent in industrial environments and may result in new products and markets for the pharmaceutical, cosmetics, toiletries, food, drink, dairy and water industries. Furthermore, handwashing and disinfection are considered to be important in reducing the likelihood of transmission of infection with many other viruses in addition to rhinoviruses and rotaviruses, and these include enteroviruses, adenoviruses, HIV, hepatitis viruses. Therefore, the novel antiviral agent can also be used as a replacement for alcohol wipes (hand disinfection in clinical situations) which are not highly thought of by nurses as they dry and damage their hands, and, on the other hand, are not very effective, since 70 - 80 % v/v ethanol only removes poliovirus type 1 Mahoney and poliovirus Sabin 1 an from hands with a mean log₁₀ reduction in plaque forming units (pfu) of 0.5 (Jones et al., 1991; Davies et al., 1993). The novel antiviral agent is also suitable to disinfect gloves, since wearing gloves protects the hands from viral contamination but changing them after each patient or contact is expensive (Davies et al., 1993).

The antiviral activity of the novel antiviral composition is preferably tested on a bacteriophage model system. The use of phages as test organisms precludes the need for a sophisticated recovery system and can be readily undertaken by any bacteriology laboratory (Davies et al., 1993). In this study, phages of both Gram positive and Gram negative were used, in particular the Pseudomonas phage NCIMB 10116 as a simple model for virucidal testing because it is resistant to most virucidal agents (Jassim et al., 1995).

Further, the composition of the present invention has also antitumor activity, i.e. it is capable of selectively killing tumor cells, whereas non-tumor cells are not significantly damaged.

The mode of action of the composition according to the present invention, e.g. the antiviral activity which extremely depends on the concentration, could not be explained yet. It was, however, found that when substituting Fe²⁺ ions (ferrous ions) by Fe³⁺ ions (ferric ions) the combination of tannic acid (0.03 % (w/v)) only showed a 2 log₁₀ reduction in the Pseudomonas phage NCIMB 10116 titer. Also, the synergistic activity of tannic acid and Fe²⁺ ions is greatly diminished when the pH of the composition is in the alkaline range. Hence, the composition of the present invention preferably has a pH of 8 or below, more preferably a pH of 7 or below. Further, it was found that the addition of detergent, e.g. 2 % (v/v) Tween 80, greatly diminished the antiviral activity. Thus, the composition of the present invention preferably is substantially free from detergent, i.e. it does not contain detergent in an antiviral activity-reducing amount. Moreover, it was found that the activity is also decreased in the absence of air/O₂. Thus, the composition of the present invention preferably contains solubilized gaseous oxygen. On the other hand, heat treatment or light treatment (irradiation) did not diminish the antiviral activity. In specific embodiments, a light treatment of the composition may be preferred.

A further aspect of the present invention is a method for controlling viruses, comprising contacting viruses with an antiviral effective amount of a composition as defined above. The contacting time can be varied in a range of from only a few seconds up to several hours. Preferably, the contacting time is from 1 min to 1 h, more preferably from 1 min to 10 min. During this time, a substantially complete killing of viruses can be obtained. The contacting temperature can also be varied within a broad range, in general from 0°C to 50°C. Generally, a temperature range from room temperature to 42°C is suitable.

In the method of the present invention the virus concentration or titer is reduced by at least the factor 10⁴, preferably by at least the factor 10⁶, more preferably by at least the factor 10⁸, still more preferably by at least the factor 10¹⁰ and most preferably by at least the factor 10¹¹ or 10¹². The reduction of virus concentration or virus titer can be measured according to known methods, e.g. as depicted in Example 1.3.

The method of the present invention is suitable for controlling viruses which are present in or on inanimate materials and/or living cells or organisms. The inanimate materials can be selected from any industrial, laboratory or hospital environments, e.g. laboratory and hospital equipment such as syringes, surfaces of laboratory or hospital facilities, food materials, dairy products, agricultural products, cosmetics, toiletries such as condoms, pharmaceutical products such as medicaments or blood products, water and drinks.

In a further embodiment the method of the present invention is suitable for treating living cells, e.g. in vitro cultivated cells such as bacterial or eukaryotic cells. Surprisingly, it was found that treating the cells with the antiviral composition of the present invention greatly reduces or even eliminates viral contamination, without causing damage to the cells.

Particularly, the present invention is suitable for controlling human and animal viruses. The composition is suitable for external use with humans and animals in the form of a liquid, e.g. as a replacement for alcohol wipes. Further, it is suitable for gynaecological applications such as decontamination of vagina and womb of women infected with herpes simplex virus in order to obviate the need of a caesarean section and/or protect the new-born babies from the infection. It is also suitable for preventing and removing initial herpes virus infections from genitals and other mucosa in immunocompromised patients, it is suitable for preventing and removing human HSV-1 which infects cells in the lining of the mouth and lips. It is further suitable for the preparation of nasal drops and/or surface disinfections to remove cold viruses of different species which invade and multiply in the cells of the nose and the throat.

Furthermore, the present invention is suitable for disinfecting dentist, surgical and barber salon tools such as combs, scissors, blades, scalpels, forceps, etc. It is suitable for disinfecting floors, walls and benches in hospital and clinical laboratories in order to control viral infections. It is suitable as a chemical coating on condoms which gives added protection from viral infection. It can be used for chemical cleaning and recycling of syringes and injection needles in order to prevent viral infections, e.g. HIV infections. There is a special need for this application in many underdeveloped parts of the world, particularly Africa and South America. It can also be used for cosmetics and toiletries industries, in water industries to control the viral infection in drinking water, cooling towers and swimming pools, and for the disinfection of sewage effluents.

The method of the present invention is also especially suitable for controlling plant viruses. The composition can be applied in liquid form to treat virus-infected plants and/or prevent plants from viral infection. The composition can also be used for treating soils, since it is a biodegradable material which enriches the soil by ions and organic materials.

A further special field of use is the decontamination of working areas in laboratories from bacteriophages which infect commercially important bacterial strains. In contrast to prior methods, the method of the present invention provides a killing of bacteriophages with no impact on the viability and survival of associated bacteria. Thus, the present invention is of great significance for the food and biotechnology industry because a differential destruction of viruses without damaging metabolically active cells such as bacteria cells which are used in the food, dairy, drink and fermentation and biotechnology industries is very important.

As described above, the composition of the present invention is suitable for use with any given carrier. The use of liquid carriers is preferred for surface detergent/disinfectant applications for laboratories, hospitals, cosmetics, toiletries, pharmaceutical products, food, drink, dairy products, fermentation processes, biotechnology industries, water and sewage industries. Further, the liquid composition is also suitable as a tool disinfectant for medical or dental uses, in barber salons etc. Further, the composition in a liquid carrier can be used as nasal drops, in syringes or needle-recycling industries, for the decontamination of nucleic acids in molecular biology techniques, e.g. nucleic acid amplification and purificiation techniques, and in agricultural applications to prevent and protect plants from virus infection.

A carrier in the form of an emulsion, e.g. a cream, is suitable for treating infections with foot-and-mouth disease viruses in animals, for treating herpes virus infections on genitals and other parts of the body, for preventing viral infection in lesion areas on human and animal skin, for treating warts, and as hand wipes for medical and research personnel carrying out work with infectious viruses.

In still another form, the composition comprises a solid carrier, e.g. a powder. Such compositions can be used e.g. in condom industries and in latex medical examination gloves industries.

Thus, the present invention also provides a pharmaceutical formulation comprising a composition as described above, optionally together with pharmaceutically acceptable adjuvants, diluents and carriers. This pharmaceutical formulation is suitable for the preparation of an antiviral agent or an antitumor agent. The pharmaceutical formulation can be used for treating living organisms such as patients in need of an antiviral or antitumor treatment.

The subject matter of the present invention is also an agricultural formulation comprising a composition as described above, optionally together with agriculturally acceptable adjuvants, diluents and carriers. This agricultural formulation can be applied to plants, agricultural food products or soils. Even an application in the field is possible.

The present antiviral composition has two main targets in viruses, i.e. virus capsid material such as proteins and lipids, and viral nucleic acid such as RNA or DNA. Surprisingly, it was found that a complete degradation of nucleic acid occurs within three minutes at room temperature. Thereby, a complete viral killing is obtained and phenomena such as multiplicity reactivation are avoided.

In a further aspect, the present invention relates to a method for degrading free or virus-associated nucleic acids, comprising contacting the nucleic acids with an nucleic acid-degradation effective amount of a composition as defined above. The method for degrading nucleic acids is suitable for treating inanimate materials and/or living cells or organisms. The contacting time and temperature are as described above.

Hence, by the present invention a method is provided, wherein free or virus-associated nucleic acids can be degraded effectively in a simple, convenient and environmentally acceptable manner. The composition and method of the present invention are especially suitable for treating materials and even living cells, where the presence of free or virus-associated nucleic acids is undesirable. One example of such materials is equipment or reagents used in molecular biological methods, e.g. in a method for the amplification of nucleic acids such as polymerase chain reaction (PCR).

A further aspect of the present invention, therefore, relates to a method for the amplification of nucleic acids, comprising decontaminating the required equipment and/or reagents with a composition as defined above before adding a sample to said equipment and/or reagents comprising the nucleic acid to be amplified. Specific examples for equipment to be decontaminated are plastic pipettes, automatic pipetters, desk tops and working areas. By the method of the present invention a complete degradation of contaminating nucleic acid molecules is obtained within a short time, whereby the need for thermal or UV-treatment is avoided. Thus, no adverse effect on the function of precision equipment such as automatic pipetters is observed.

Preferably, the decontamination agent according to the present invention is neutralized before the sample is added. This neutralization can be carried out by adding a detergent, preferably a non-ionic detergent such as Tween 80 and/or by adjusting an alkaline pH.

Furthermore, the present invention is illustrated by the enclosed figures.
- Fig. 1: shows the effect of different concentrations of tannic acid and 3.15 mmol/l ferrous sulfate on the survival of Pseudomonas phage NCIMB 10116 at room temperature for 3 min.
- Fig. 2: shows the susceptibility of MT4 cells to HIV-1 infection; A: treatment with antiviral agent (0.03 % (w/v) tanpic acid and 3.15 mmol/l ferrous sulfate) at a 1:1 dilution with λ-buffer; B: treatment with undiluted antiviral agent; C and D: controls treated with λ-buffer.
- Fig. 3: shows the results of a test for the presence of potato leafroll virus by ELISA. Columns 8 and 10, lines E, F, G and H are samples treated with antiviral agent. Column 11, lines E, F, G and H are samples without treatment (all readings above 10 were regarded as virus positive).
- Fig. 4: shows the results of a gel electrophoresis determining phage DNA degradation. Lane 1: 1 Kb DNA marker; lane 2: E.coli phage ARI untreated; lane 3: E.coli phage ARI treated with antiviral agent; lane 4: E.coli phage ARI lysed; lane 5: E.coli phage ARI DNA; lane 6: E.coli phage ARI DNA treated with antiviral agent; lane 7: E.coli plasmid DNA; lane 8: E.coli plasmid DNA treated with antiviral agent; lane 9: 1Kb marker; lane 10: λ/Hind III marker.
- Fig. 5: shows the results of a gel electrophoresis measuring the amplification products from a PCR. Lane 1: λ/Hind III marker; lane 2: β-actin amplified genomic DNA untreated; lane 3: β-actin amplified genomic DNA treated with antiviral agent.
- Figs. 6A and 6B: show transmission electron photomicrographs (Mag. x 288000) of Pseudomonas phage NCIMB 10116 before treatment with antiviral agent (Fig. 6A) and after treatment with antiviral agent (Fig. 6B)

### Examples

### 1. Materials and method

### 1.1. Bacteria and phage strains

Listeria monocytogenes ATCC 23074, Staphylococcus aureus ATCC 27694, Pseudomonas aeruginosa NCIMB 10548 and Salmonella typhimurium ATCC 19585 bacteria were used. The phages with specificity for the above bacteria were ATCC 23074-B1 for L. monocytogenes, ATCC 27694-B1 for Staph. aureus, NCIMB 10116 for Ps. aeruginosa and ATCC 19585-B1 for S. typhimurium.

The bacterial cells were maintained on Tryptose Phosphate Broth (TPB; Oxoid) supplemented with 1 % agar (TPA) and stored at 4°C with monthly subculture. When required, cells were resuscitated in 10 ml TPB (18 h, 37°C) on an orbital shaker operating at 240 rpm. Appropriate bacterial dilutions were made in Lambda buffer (6 mmol/l Tris, 10 mmol/l MgSO₄·7H₂O, 50 µg/ml gelatin; pH 7.2). After treatment, bacterial survival was determined by colony forming units (cfu) on TPA according to Miles et al. (1938).

Phage stocks were developed on their appropriate host strains by a plate lysis procedure essentially equivalent to growing phage Lambda-derived vectors (Ausubel et al., 1991). Typical phage titers of 10¹⁰ pfu/ml were obtained. Phage stocks were maintained in Lambda buffer at 4°C and stocks retained a constant titer for several months.

### 1.2 Preparation of tannic acid

Formulations of tannic acid and some tannic acid derivatives were prepared to investigate their antiviral activity as follows.

### 1.2.1. Untreated tannic acid

A stock solution (10 % w/v) of tannic acid (Sigma, CAS 1401-55-4, extracted from oak gall nut (Quercus infectoria) was prepared in distilled water and stored at room temperature.

### 1.2.2. Heat-treated tannic acid

A solution of tannic acid (0.50 gM, Sigma) in 45 ml of methanol was heated at reflux (65°C) for 20 h. The reaction was cooled to room temperature and the solvent removed in vacuo. This afforded a solid which was then used in the testing for activity.

### 1.2.3. Light-treated tannic acid

A solution of tannic acid (0.50 gM, Sigma) in 30 ml of methanol was degassed with argon for 10 min. The solution was then irradiated (Hanovia 450 W medium pressure mercury vapor lamp) through Pyrex at 0°C for 2 h. The solvent was removed in vacuo to afford a solid that was used in the testing for activity.

### 1.2.4. Degassing tannic acid

A solution of tannic acid (0.5 gM, Sigma) in 50 ml distilled water was degassed by the freeze-pump-thaw method which involved cooling the samples in a -78°C bath, pumping on the cooled sample at about 1 mm Hg venting the vacuum to argon and allowing the sample to thaw. This procedure was twice repeated. After three cycles of the freeze-pump-thaw sequence the solution was kept under argon and used in the testing for activity.

### 1.2.5. Solid tannic acid formulation

A dry formulation of tannic acid was prepared by mixing 30 mg tannic acid with 70 mg FeSO₄·7H₂O in a dark container. The dry mixture could be stored at room temperature in a dark container for over one year. Before use, 100 ml buffer or distilled water was added to the powdered mixture.

### 1.3. Virucidal assay

Immediately prior to use, the stock solution of tannic acid was diluted in Lambda buffer to obtain desired concentrations (0.01, 0.1, 1 and 3 %). A 300 µl sample of an appropriate dilution of tannic acid was added to 700 µl of preferably freshly prepared ferrous sulfate solution in lambda(λ)-buffer (4.5 mmol/l FeSO₄·7H₂O; pH 6.5). Thereby tannic acid concentrations of 0.003, 0.03, 0.3 and 0.9 % (w/v) and ferrous sulfate concentrations of 3.15 mmol/l in the final composition were obtained.

These mixtures were protected from light. Phage (20 µl at 10¹⁰ pfu/ml) or 20 µl of an appropriate dilution of bacteria (10⁹ cfu/ml) were placed in a sterile Eppendorf micro-centrifuge tube and 150 µl of the tannic acid/FeSO₄·7H₂O mixture were added. After exposure of the phage or bacteria for 3 min at room temperature the activity of the mixture was neutralized by adding an equal volume of 2 % (v/v) Tween 80 in Lambda buffer. The number of phages or bacteria surviving the above protocol was measured by plaque forming units (pfu) or colony forming units (cfu), respectively.

### 2. Results

### 2.1. The prokaryotic viruses

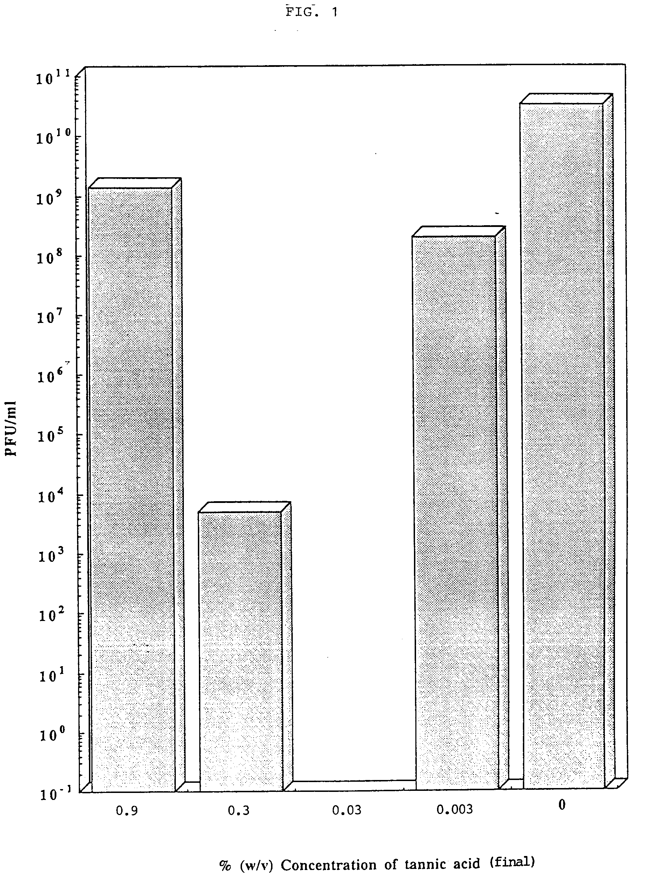

Table 1 indicates that all concentrations of tannic acid alone have a slight virucidal activity resulting in only one log₁₀ reduction in plaque forming ability, against Pseudomonas phage NCIMB 10116. However, the presence of Fe²⁺ ions enhances the virucidal activity and only about 0.03 % of tannic acid in combination with FeSO₄7H₂O gives a profound synergy producing ten log₁₀ reductions in plaque forming ability within 3 min. Above and below 0.03 % tannic acid the synergistic effect of ferrous sulfate was reduced (see Table 1 and Fig. 1). Neither heat nor light treatment of tannic acid eliminates the antiviral activity when they are mixed with ferrous sulfate, whereas the antiviral activity of the combination was diminished or even abolished at alkaline pH, tannic acid and ferrous sulfate prepared under degassing conditions and by the addition of 2 % (v/v) Tween 80.

A substitution of tannic acid by ellagic acid (4,4',5,5',6,6'-hexahydroxydiphenic acid 2,6,2',6'-dilactone, C₁₄H₆O₈ FW 302.2) (3.5 % (w/v), Sigma, isolated from tannin extracts), gallic acid (3,4,5-trihydroxybenzoic acid, C₇H₆O₅ FW 170.1) (0.5 % (w/v), Sigma) or hydroquinone (1,4-benzenediol, C₆H₆O₂ FW 110.1 (0.5 and 5 % (w/v), Sigma) alone or in combination with ferrous ions (3.15 mmol/l) all failed to inactivate Pseudomonas phage NCIMB 10116.

It was found that ferrous salts (3 to 6 mmol/l) whether FeSO₄·7H₂O or (NH₄)₂ SO₄·FeSO₄ only in combination with 0.03 % (w/v) tannic acid are capable of reducing the Pseudomonas phage NCIMB 10116 titer by > 10 log₁₀ in less than 3 min at room temperature, whereas FeCl₃ (3.15 mmol/l), ZnSO₄·7H₂O (3.15 mmol/l) in combination with the tannic acid reduced the phage titer by only 2 to 4 log₁₀, respectively, after 5 min incubation at room temperature. CuSO₄·7H₂O (3.15 mmol/l) alone or in combination with the tannic acid gives no antiviral activity (phage titer did not reduce even after 30 min contact time with the antiviral agent at room temperature).

The combination of 0.03 % tannic acid and ferrous sulfate (3.15 mmol/l) has no effect on the survival of Gram negative bacteria for up to 120 min at room temperature (cf. Table 2). Though the Gram positive bacteria L. monocytogenes ATCC 23074 and Staph. aureus ATCC 27694 found susceptible to the antiviral agent after 120 min incubation, the bacterial titer was only reduced by 2 log₁₀ at room temperature.

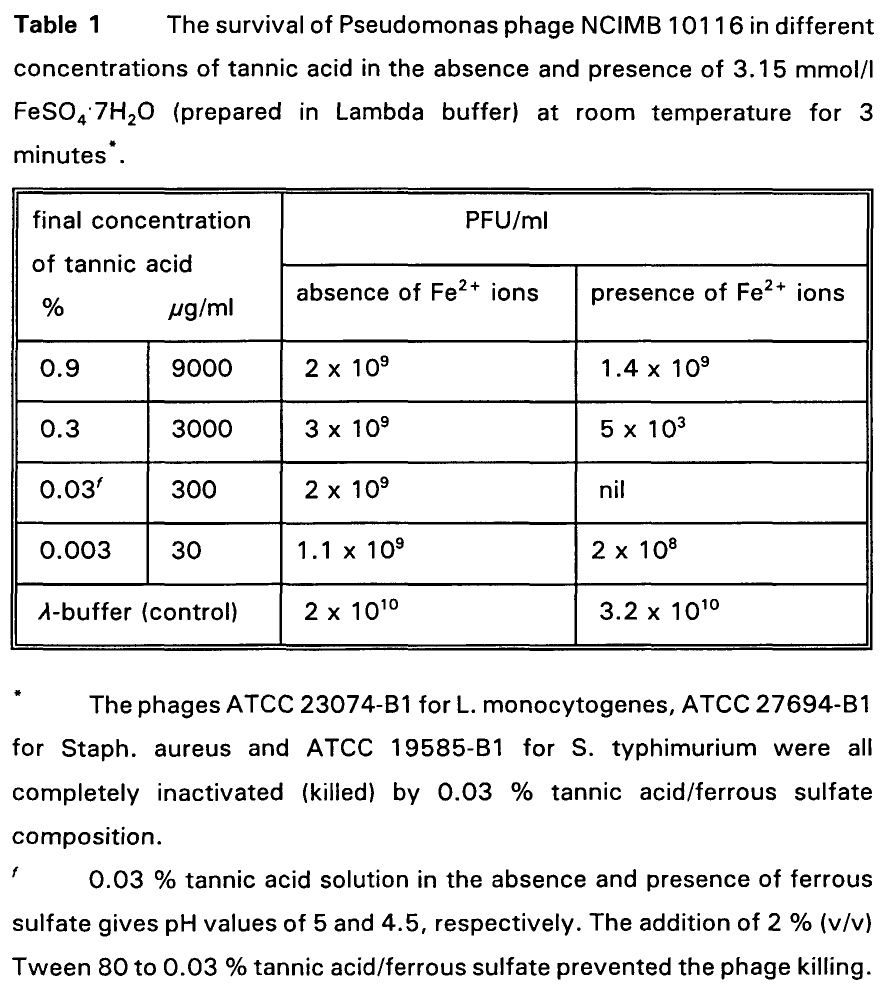

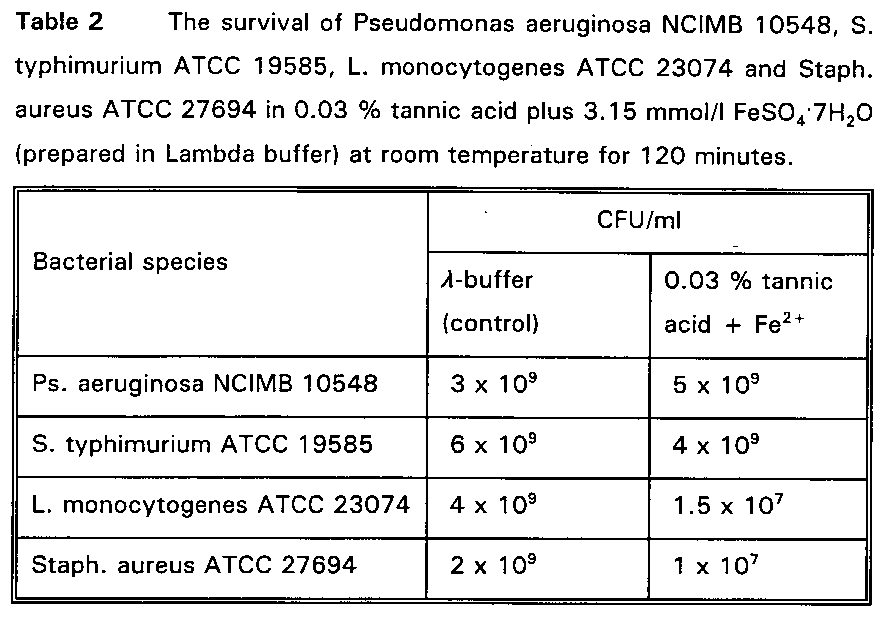

### 2.2. The eukaryotic viruses

Work was carried out to investigate the effect of the antiviral composition comprising 0.03 % tannic acid and 3.15 mmol/l ferrous sulfate on eukaryotic cell viruses.

### 2.2.1. Human viruses

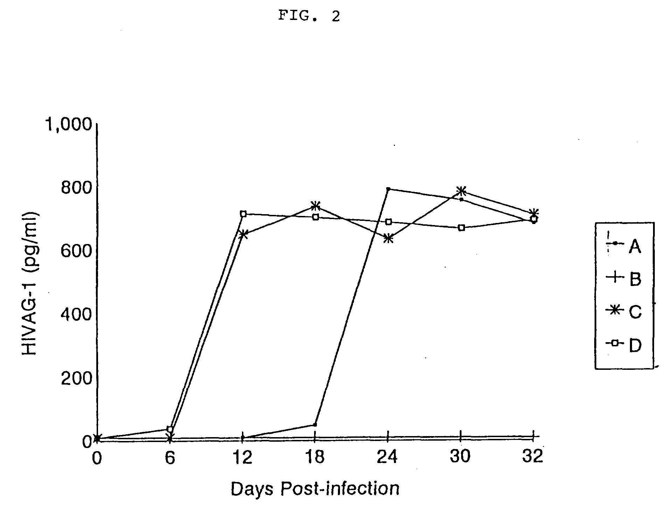

A test showed that the novel antiviral agent at the normal concentration (0.03 % (w/v) tannic acid and 3.15 mmol/l ferrous sulfate) completely inhibited the growth of HIV-1 after 3 min treatment in the presence of MT4 cells, and no growth was observed after 32 days of the post-infection (cf. Fig. 2). It was noticed that there was a delay (approximately 18 days) in the amplification of the HIV-1 when a half concentration (1:1 normal concentration: λ-buffer) of the antiviral agent mixture was used. In comparison thereto, in the absence of the antiviral agent mixture there was a normal, slow growth of HIV-1 which started after 6 days in the presence of λ-buffer as control.

Further experiments showed that the herpes simplex HSV-1 and HSV-2 and influenza viruses were all killed within 3 min contact time with the novel antiviral agent.

### 2.2.2. The animal viruses

The effect of the novel antiviral agent on the plaque formation of fowlpox virus on chicken embryo fibroblast cells was tested. Fowlpox virus was used with a titer of 1 x 10⁵ pfu/ml. Cells used were monolayers of primary chicken embryo fibroblast cultures in 60 mm diameter dishes.

10 µl of virus solution was taken and 150 µl antiviral agent was added. After incubation for 5 min at room temperature, 150 µl neutralizing material (2 % (v/v) Tween 80 in λ-buffer) was added. The titer of the virus was determined by plaque titration according to standard virological methods. The plaques were visualized by neutral red, counted and recorded. Controls included: virus without treatment; 10x dilution of the virus treated with antiviral agent; virus treated with neutralizing agent only.

No plaques (indicative of virus replication) were observed where the treated virus was plated at any dilution. The control samples (virus untreated or treated with neutralizing material), on the contrary, showed no reduction in virus titer.

### 2.2.3. The plant viruses

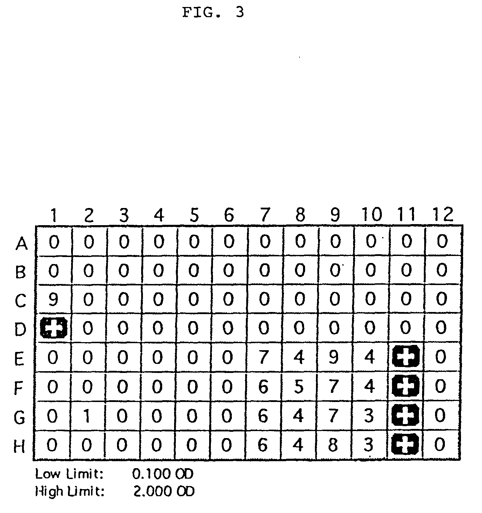

The effect of the novel antiviral agent on the potato leafroll virus was tested in the field and in the laboratory. In the field, 10 potato plants infected with leafroll virus received single treatment with the novel antiviral agent (0.03 % (w/v) tannic acid and 3.15 mmol/l Fe²⁺) using the spray method. At the same time, another 10 plants infected with the virus received single treatment with water spray as control samples. After 24 hours leaves were harvested and collected in individual bags. In the laboratory, the leaves were washed with distilled water, ground and the syrup was tested for the presence of the virus using ELISA technique. The results obtained with the Macintosh Microplate Manager II system (Fig. 3) clearly demonstrated that virus replication significantly decreased after a single treatment with the antiviral agent as compared with the plants treated with water only.

Similarly, other plant viruses, e.g. wheat streak mosaic virus, luteovirus, tomato virus, were all killed after a single field treatment.

### 2.3. Nucleic acid degradation

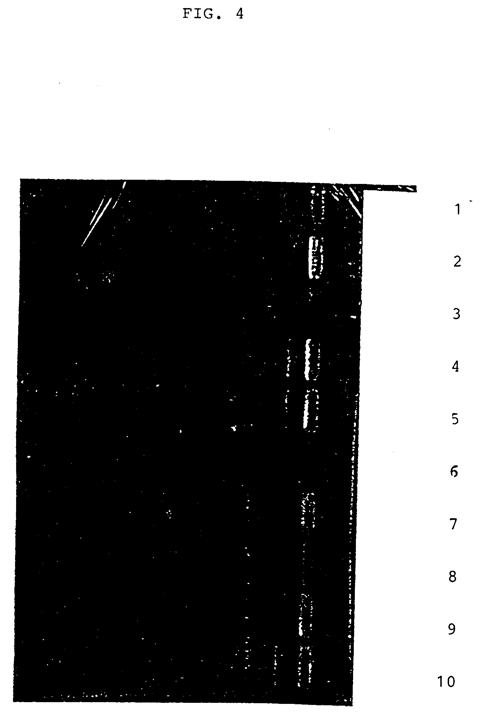

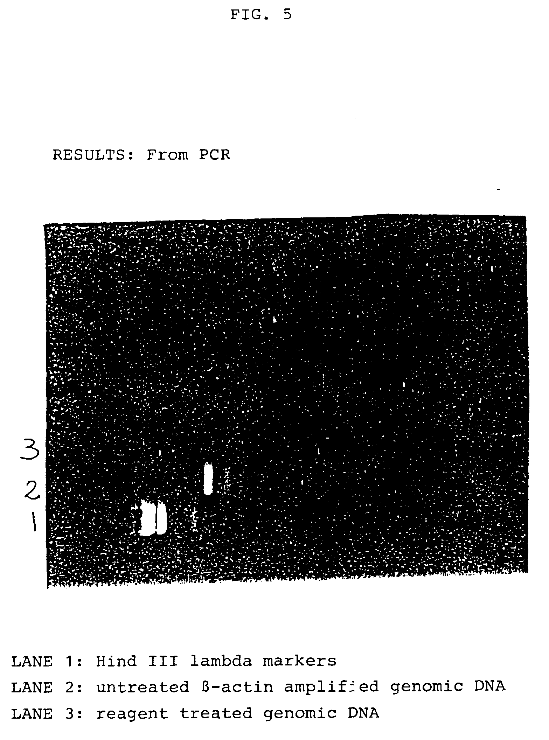

To determine the phage DNA degradation, gel electrophoresis (see Fig. 4) of supernatants from Escherichia coli phage ARI, phage ARI DNA, and plasmid E. coli treated with the virucidal agent was carried out. It was found that complete degradation had occurred after 3 min treatment, followed by the addition of 2 % (v/v) Tween 80 as neutralizer for the virucidal agent. Comparative samples treated with λ-buffer and 2 % (v/v) Tween 80 as controls, however, exhibited normal DNA band patterns. Furthermore, the results from a PCR-based method also confirmed that the genomic DNA had degraded completely after 3 min treatment with the combination of the virucidal agent of tannic acid (0.03 % (w/v)) and FeSO₄·7H₂O (3.15 mmol/l) solution (cf. Fig. 5).

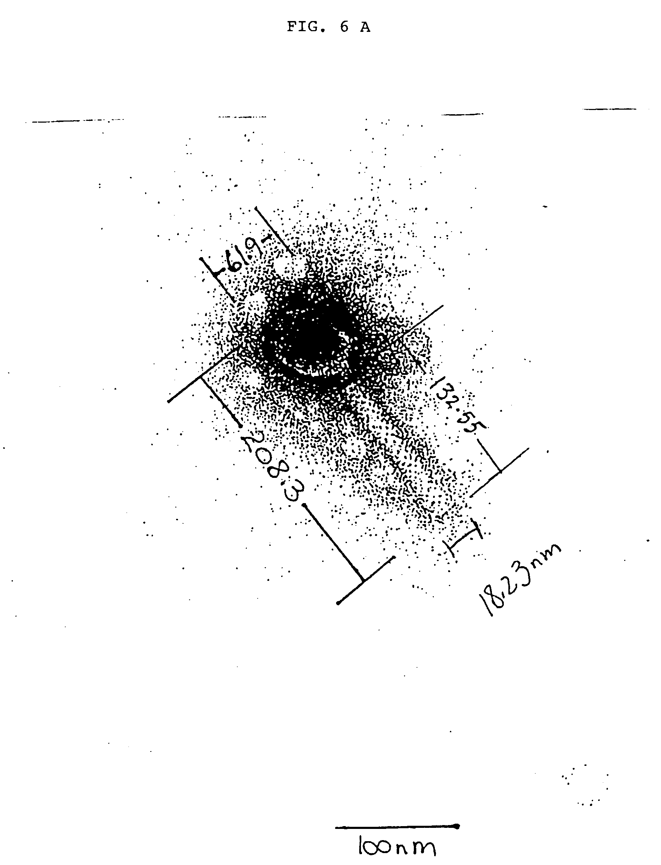

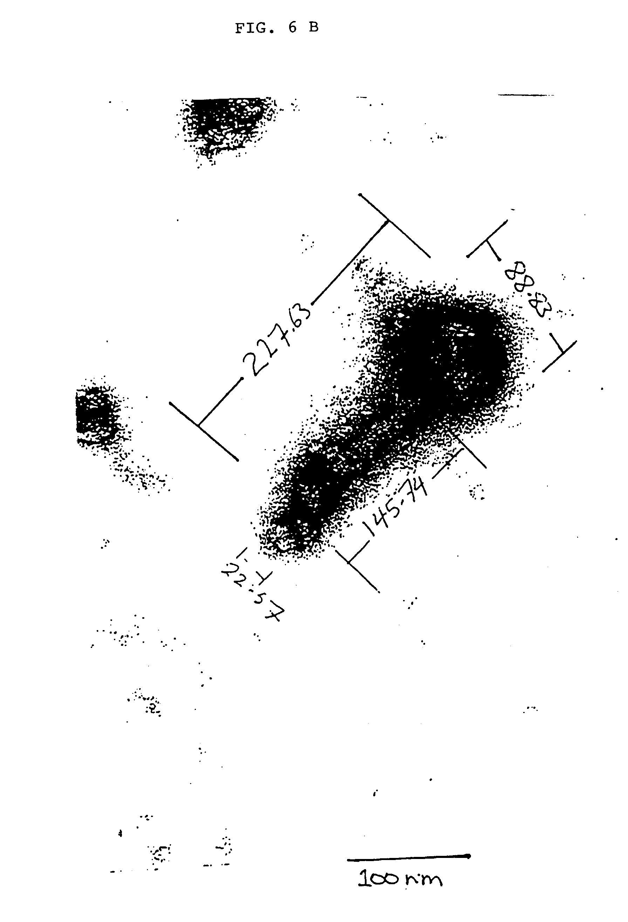

The transmission electron photomicrographs at 288000x magnification, of the treated and non-treated (treated with λ-buffer) Pseudomonas phage NCIMB 10116 with the combination of tannic acid (0.03 % (w/v)) and FeSO₄·7H₂O (3.15 mmol/l) solution, (cf. Figs. 6a and 6b) revealed that the Pseudomonas phage had swollen and increased in size as compared to the non-treated phage.

By the treatment the overall length of the virus particle increased from 208.3 nm to 227.63 nm. The capsid diameter showed an increase from 61.9 to 88.8 nm. The virus tail showed an increase from 132.55 to 145.74 nm.

### References

Abad, F.X., Pinto, R.M. and Bosch, A. (1994), Survival of enteric viruses on environmental fomites, Appl. Environ. Microbiol. 60, 3704-3710.
Adams, M.H. (1959), Bacteriophages, pp. 49-62, Wiley Interscience.
Araujo, R.M., Puig, A., Lasobras, J., Lucena, F. and Jofre, J. (1997), Phages of enteric bacteria in fresh water with different levels of faecal pollution, J. Appl. Microbiol. 82, 281-286.
Armon, R. and Kott, Y. (1995), Distribution comparison between coliphages and phages of anaerobic bacteria (Bacteroides fragilis) in water sources, and their reliability as fecal pollution indicators in drinking water, Water Sci. Technol. 31, 215-222.
Ausubel, F.M., Brent, R., Kingston, R.E., Moore, D.D., Seidman, J.G., Smith, J.A. and Struhl, K. (1991), Growing lambda-derived vectors in current protocols in molecular biology, Vol. 1, pp. 1.12.1 - 1.12.3, Wiley Interscience, New York.
Bosh, A., Pinto, R.M. and Abad, F.X. (1995), Differential accumulation and depuration of human enteric viruses by mussels, Water Sci. Technol. 31, 447-451.
Carson, C.F. and Riley, T.V. (1992), Antimicrobial compounds from south western Australian plants, Austral. Microbiol. 13, No. 3, A126.
Chantrill, B. H., Coulthard, C.E., Dickinson, L., Inkley, G.W., Morris, W. and Pyle, A.H. (1952), The action of plant extracts on a bacteriophage of Pseudomonas pyocyanea and on influenza A virus, J. Gen. Microbiol. 6, 74-84.
Davidson, P.M. and Branden, A.L. (1981), Antimicrobial activity of non-halogenated phenolic compounds, J. Food Protect. 44, 623-632.
Davies, J.G., Babb, J.R., Bradley, C.R. and Ayliffe, G.A.J. (1993), Preliminary study of test methods to assess the virucidal activity of skin disinfectants using poliovirus and bacteriophages, J. Hosp. Infect. 25, 125-131.
Dutka, B.J., Palmateer, G.A., Meissner, S.M., Janzen, E.M. and Sakellaris, M. (1990), The presence of bacterial virus in groundwater and treated drinking water, Environ. Pollut. 63, 293-298.
Evans, W.C. (1989), Trease and Evan's Pharmacognosy, Baillière Tindall.
Fujita, H., Endo, A. and Suzuki, K. (1981), Inactivation of bacteriophage lambda by near-ultraviolet irradiation in the presence of chlorpromazine, Photochem. Photobiol. 33, 215-222.
Fujita, H. and Matsuo, I. (1987), Phototoxic potential of afloqualone, a quinazolinone derivative, as determined by photosensitized inactivation of bacteriophage, Chem. Biol. Interactions. 64, 139-149.
Fukuchi, K., Sakagami, H., Okuda, T., Hatano, T., Tanuma, S.-I., Kitajima, K., Inoue, Y., Inoue, S., Ichikawa, S., Nonoyama, M. and Konno, K. (1989), Antiviral Res. 11, 285.
Gordon, V., Parry, S., Bellamy, K. and Osborne, R. (1993), Assessment of chemical disinfectants against human immunodeficiency virus: overcoming the problem of cytotoxicity and the evaluation of selected actives, J. Virol. Methods. 45, 247-257.
Haslam, E. (1989), Plant polyphenols, Cambridge University Press, Cambridge.
Havelaar, A. H., Vanolphen, M. and Schijven, J.F. (1995), Removal and inactivation of viruses by drinking-water treatment processes under full-scale conditions, Water Sci. Technol. 31, 55-62.
Hibma, A.M., Jassim, S.A.A., Griffiths, M.W. (1997), Infection and removal of L-forms of Listeria monocytogenes with bred bacteriophage, Int. J. Food Microbiol. 34, 197-207.
Hirotani, H., Ohigashi, H., Kobayashi, M., Koshimizu, K. and Takahashi, E. (1991), Inactivation of T5 phage by cis-vaccenic acid, an antivirus substance from Rhodopseudomonas capsulate, and by unsaturated fatty acids and related alcohols, FEMS Microbio. Letts. 77, 13-18.
Hudson, J.B. (1990), Antiviral compounds from plants, CRC Press, Boca Raton.
Jassim, S.A.A., Akoush, S. and Griffiths, M.W. (1996), Rapid detection using thermal change to monitor infection by host specific bacteriophage, IUFOST Meeting Food Associated Pathogens, Uppsala, Sweden, May 1996.
Jassim, S.A.A., Ellison, A., Denyer, S.P. and Stewart, G.S.A.B. (1990), In vivo bioluminescence: a cellular reporter for research and industry, J. Biolumin. Chemilumin. 5, 115-122.
Jassim, S.A.A., Stewart, G.S.A.B. and Denyer, S.P. (1995), Antiviral or antifungal composition and method, International Patent Application No. WO 95/22254.
Jassim, S.A.A., Stewart, G.S.A.B., Denyer, S.P., Rostas-Mulligan, K. and Ress, C.E. (1992), Methods for rapid microbial detection, International Patent Application No. WO 92/02633.
Jofre, J., Olle, E., Lucena, F. and Ribas, F. (1995a), Bacteriophage removal in water-treatment plants, Water Sci. Technol. 31, 69-73.
Jofre, J., Olle, E., Ribas, F., Vidal, A. and Lucena, F. (1995b), Potential usefulness of bacteriophages that infect Bacteroides fragilis as model organisms for minitoring virus removal in drinking treatment plants, Appl. Environ. Microbiol. 61, 3227-3231.
Jones, M.V., Bellamy, K., Alcock, R. and Hudson, R. (1991), The use of bacteriophage MS2 as a model system to evaluate virucidal hand disinfectants, J. Hosp. Infect. 17, 279-285.
Kodikara, C.P., Crew, H.H. and Stewart, G.S.A.B. (1991), Near on-line detection of enteric bacteria using lux recombinant bacteriophage, FEMS Microbiol. Letts. 83, 261-266.
Konowalchuk, J. and Speirs, J.I. (1976a), Virus inactivation by grapes and wines, Appl. Environ. Microbiol. 32, 757-763.
Konowalchuk, J. and Speirs, J.I. (1976b), Antiviral activity of fruit extracts., J. Food Sci. 41, 1013-1017.
Konowalchuk, J. and Speirs, J.I. (1978a), Antiviral effect of commercial juices and beverages, Appl. Environ. Microbiol. 35, 1219-1220.
Konowalchuk, J. and Speirs, J.I. (1978b), Antiviral effect of apple beverages, Appl. Environ. Microbiol. 36, 798-801.
Kott, Y., Roze, N., Sperber, S. and Betzer, N. (1974), Bacteriophages as viral pollution indicators, Water Research 8, 165-177.
Maillard, J.-Y., Beggs, T.S., Day, M.J., Hudson, R.A. and Russell, A.D. (1993), Effect of biocides on Pseudomonas aeruginosa phage F116, Letts. Appl. Microbiol. 17, 167-170.
Maillard, J.-Y., Beggs, T.S., Day, M.J., Hudson, R.A. and Russell, A.D. (1996a), The effect of biocides on proteins of Pseudomonas aeruginosa PAO1 bacteriophage F116, J. Appl. Bacteriol. 80, 291-295.
Maillard, J.-Y., Beggs, T.S., Day, M.J., Hudson, R.A. and Russell, A.D. (1996b), Damage to Pseudomonas aeruginosa PAO1 bacteriophage F116 DNA by biocides, J. Appl. Bacteriol. 80, 540-544.
Maillard, J.-Y., Hann, A.C., Beggs, T.S., Day, M.J., Hudson, R.A. and Russell, A.D. (1995), Electron microscopic investigation of the effects of biocides on Pseudomonas aeruginosa PAO bacteriophage F116, Journal of Medical Microbiology 42, 415-420.
Martin, S.J. (1978), The biochemistry of viruses, pp. 121-124, Cambridge University Press.
Mesquita, M.M.F.D. (1990), Bacteriophages as viral models in studies of water and shellfish quality, Rev. Bras. Biol. 49, 923-932.
Miles, A.A., Misra, S.S. and Irwin, J.O. (1938), The estimation of the bactericidal power of the blood, J. Hyg. (Cambridge) 38, 732-749.
Phillips, J.D. (1992), Medicinal plants, J. Instit. Biolog. 39 (5), 187-191.
Quinn, P.J. (1992), Virucidal activity of disinfectants, in Principles and practice of disinfection, preservation and sterilization, 2nd edn., eds. Russell, A.D., Hugo, W.B. and Ayliffe, G.A.J., pp. 150-170, Oxford: Blackwell Science.
Sagripanti, J.-L., Routson, L.B. and Lytle, C.D. (1993), Virus inactivation by copper or iron ions alone and in the presence of peroxide, Appl. Environ. Microbiol. 59, 4374-4376.
Shead, A., Vickery, K., Medhurst, R. and Freiman, J. (1992), Neutralisation but not cure of duck hepatitis B by Australian Phyllanthus extracts, Austral. Microbiol. 13, No. 3, A111.
Stewart, G.S.A.B., Smith, A.T. and Denyer, S.P. (1989), Genetic engineering of bioluminescent bacteria, Food Sci. Technol. Today 3, 19-22.
Suzuki, K., Fujita, H., Yanagisawa, F. and Kitakami, M. (1977), Action spectrum for the photo-inactivation of lambda and T4 bacteriophages sensitized with 8-methoxypsoralen, Photochem. Photobiol. 26, 49-51.
Tartera, C., Bosch, A. and Jofre, J. (1988), The inactivation of bacteriophages infecting Bacteroides fragilis by chlorine treatment and UV-irradiation, FEMS Microbiol. Lett. 56, 313-316.
Thraenhart, O., Kuwert, E.K., Dermietzel, R., Kuwert, E., Scheiermann, N. and Wendt, F. (1978), Influence of different disinfection conditions on the structure of the Hepatitis B viurs (Dane particle) as evaluated in the morphological alteration and disintegration test (MADT), Zentralblatt für Bakteriologie, Parasitenkunde, Infektionskrankheiten und Hygiene, I. Abteilung Originale, Reihe B 160, 541-550.
Thresh, J.M. (1956), Some effects of tannic acid and of leaf extracts which contain tannins on the infectivity of tobacco mosaic and tobacco necrosis viruses, Ann. App. Biol. 44, 608-618.
Thurman, R.B. and Gerba, C.P. (1989), The molecular mechanisms of copper and silver ion disinfection of bacteria and viruses, Critical Reviews in Environmental Control 18, 295-315.
Turpin, P.E., Maycroft, K.A., Bedford, J., Rowlands, C.L. and Wellington, E.M.H. (1993), A rapid luminescent-phage based MPN method for the enumeration of Salmonella typhimurium in environmental samples, Letts. Appl. Microbiol. 16, 24-27.
Ulitzur, S. and Kuhn, J. (1987), Introduction of lux genes into bacteria, a new approach for specific determination of bacteria and their antibiotic susceptibility, in Bioluminescence and Chemiluminescence New Perspectives, (Schlomerich, J., Andereesen, R., Kapp, A., Ernst, M. and Woods, W.G., Eds.), pp. 463-472, Bristol: Wiley.
Wolber, P.K. and Green, R.L. (1990), New rapid method for the detection of Salmonella in foods, Trends Food Sci. Technol. 1, 80-82.
Young, D.C. and Sharp, D.G. (1985), Virion conformational forms and the complex inactivation kinetics of echovirus by chlorine in water, Applied and Environmental Microbiology 49, 359-364.

## Claims

1. A composition comprising a carrier and an active agent which is a combination of (a) tannic acid or components thereof in a concentration equivalent to 0.003 - 0.3 % (w/v) or in a concentration equivalent to from 30 to 3000 µg/ml tannic acid from Quercus infectoria and (b) a source of Fe²⁺ ions.

2. The composition of claim 1 comprising tannic acid or components thereof in a concentration equivalent to 0.015 - 0.15 % (w/v) tannic acid from Quercus infectoria.

3. The composition of claim 2 comprising tannic acid or components thereof in a concentration equivalent to about 0.03 % (w/v) tannic acid from Quercus infectoria.

4. The composition of any one of claims 1 to 3, wherein the active agent is a tannic acid from Quercus infectoria or any other plant.

5. The composition of any one of claims 1 to 4, wherein the source of Fe²⁺ ions is a Fe-(II)-salt.

6. The composition of claim 5, wherein the Fe-(II)-salt is selected from Fe-(II)-sulfate, Fe-(II)-halides and organic Fe-(II)-salts.

7. The composition of any one of claims 1 to 6 having antiviral activity.

8. The composition of claim 7 having antiviral activity against viruses selected from bacteriophages, animal viruses and plant viruses.

9. The composition of claim 8 having activity against human viruses.

10. The composition of any one of claims 1 to 6 having antitumor activity.

11. Pharmaceutical formulation comprising a composition of any one of claims 1 to 10 optionally together with pharmaceutically acceptable adjuvants, diluents and carriers.

12. Use of a pharmaceutical formulation of claim 11 for the preparation of an antiviral agent.

13. Use of a pharmaceutical formulation of claim 11 for the preparation of an antitumor agent.

14. Agricultural formulation comprising a composition of any of claims 1 to 10, optionally together with agriculturally acceptable adjuvants, diluents and carriers.

15. A method for controlling viruses, comprising contacting viruses with an antiviral effective amount of a composition of any one of claims 1 to 11 or 14.

16. The method of claim 15, wherein the contacting time is from 1 min to 1 h.

17. The method of claim 15, wherein the contacting temperature is from 0°C to 50°C.

18. The method of any one of claims 15 to 17, wherein the virus concentration is reduced by at least the factor 10⁴.

19. The method of any one of claims 15 to 18, comprising contacting inanimate materials and/or living cells or organisms.

20. The method of claim 19, wherein said materials are selected from laboratory and hospital equipment, surfaces of laboratory or hospital facilities, food, dairy products, cosmetics, toiletries, pharmaceutical products, water, drinks and agricultural products.

21. The method of claim 19, wherein said cells are in vitro cultivated cells.

22. The method of claim 19, wherein said organisms are patients in need of an antiviral treatment.

23. The method of claim 19, wherein said organisms are plants.

24. The method of claim 23, wherein said plants are contacted in the field.

25. A method for degrading free or virus-associated nucleic acids, comprising contacting the nucleic acids with a nucleic acid degradation effective amount of a composition of any one of claims 1 to 11 or 14.

26. The method of claim 25 comprising contacting inanimate materials and/or living cells or organisms.

27. A method for the amplification of nucleic acids, comprising decontaminating the required equipment and/or reagents with a composition of any one of claims 1 to 11 before adding a sample comprising the nucleic acid to be amplified.

28. The method of claim 27 further comprising neutralizing the decontamination agent before adding the sample.

29. The method of claim 28, wherein said neutralizing is carried out by adding a detergent and/or by adjusting an alkaline pH.
